# EUROPEAN PATENT SPECIFICATION

(11) **EP 2 615 985 B1**
(45) Date of publication and mention of the grant of the patent: **22.07.2020**
(21) Application number: 11760929.7
(22) Date of filing: 13.09.2011
(51) Int. Cl.: A61B 17/072, A61B 1/00, A61B 1/313, A61B 17/00, A61B 17/29, A61B 17/32, H01M 2/10, H02J 7/00

(54) **SURGICAL INSTRUMENTS AND BATTERIES FOR SURGICAL INSTRUMENTS**
CHIRURGISCHE INSTRUMENTE UND BATTERIEN FÜR CHIRURGISCHE INSTRUMENTE
INSTRUMENTS CHIRURGICAUX ET BATTERIES POUR INSTRUMENTS CHIRURGICAUX

(30) Priority: 17.09.2010 US 884838
(43) Date of publication of application: 24.07.2013
(73) Proprietor: Ethicon Endo-Surgery, Inc., Cincinnati, OH 45242 (US)
(72) Inventor: SHELTON IV, Frederick, E., Hillsboro, OH 45133 (US); SCHWEMBERGER, Richard, F., Cincinnati OH 45247 (US); ABBOT, Daniel, J., Loveland OH 45140 (US); SMITH, Bret, W., Kings Mills OH 45034 (US); YATES, David, C., West Chester OH 45069 (US); SCHLEITWEILER, Patrick, M., West Chester OH 45069 (US); BOURDREAUX, Chad, B., Cincinnati, OH 45242 (US)
(74) Representative: Tunstall, Christopher Stephen
(86) International application number: PCT/US2011/051366
(87) International publication number: WO 2012/037103

(56) References cited:
- EP-A1- 0 528 478
- US-A- 5 868 790
- US-A1- 2008 123 262
- US-A1- 2009 057 369
- US-A1- 2009 090 763
- US-A1- 2010 076 475
- US-B2- 6 482 200
- US-B2- 7 631 981

## Description

### BACKGROUND

A growing number of surgical instruments are powered by one or more battery cells. Such instruments include a variety of electrically powered implements and may be used in a variety of surgical environments. For example, battery-powered surgical instruments may include motor-driven implements (cutters, graspers, staplers, *etc.*) and/or non-motor driven implements (*e.g.,* RF cutter/coagulators, ultrasonic cutter/coagulators, laser cutter/coagulators, *etc*.)*.* Battery-powered instruments are also used now in various different surgical environments including, for example, endoscopic environments, laparoscopic environments, open environments, *etc.*

Battery-powered surgical instruments often utilize primary cells, which are precharged and often intended for a single discharge (*e.g.,* one use). This avoids the difficulties associated with re-sterilizing and recharging secondary, rechargeable cells. Primary cells, however, present additional challenges related to shipping, storage and disposal.

US 2009/057369 A1 relates to an electrically operated surgical instrument that includes a surgical end effector having an actuation assembly effecting a surgical procedure when actuated and a handle connected to the end effector for actuating the assembly. A part of the assembly moves between start and fully actuated positions. The handle has a self-contained power supply and a drive assembly disposed entirely within the handle.

US 5868790 A relates to a battery pack for a portable defibrillator. The battery pack has a latch that is biased in an extended position. When the battery pack is inserted into a battery well in the portable defibrillator, the latch automatically latches into a slot to secure the battery pack in the battery well.

US 7631981 B2 relates to a disposable light for use with a speculum. Generally, the disposable light includes a casing with an interior and an exterior. The casing interior may be adapted to hold at least one battery. The casing exterior may include a proximal surface and a distal surface.

### SUMMARY

There is provided a surgical system according to claim 1. Preferred embodiments are defined in the dependent claims.

A second battery pack may be positioned within the second cavity and may be in electrical contact with at least one of the handle and the end effector. The second battery pack may comprise: a second casing having a cross-sectional shape corresponding to the second asymmetrical cross-sectional shape, and a second plurality of cells electrically coupled to one another and positioned within the second casing.

Also, various embodiments may be directed to a surgical system comprising a battery pack. The battery pack may comprise a casing and a plurality of cells positioned within the casing. At least a portion of the plurality of cells may not be electrically connected to one another. The battery pack may also comprise a first switch having an open position and a closed position. In the closed position, the first switch may electrically interconnect the plurality of cells. The first switch may be mechanically biased to the open position. The battery pack may further comprise a discharge switch having an open position and a closed position. The discharge switch may be positioned to, when in the closed position, electrically connect an anode of the battery pack to a cathode of the battery pack. The discharge switch may be mechanically biased to the closed position, and may be held in the open position by a portion of the casing.

According to various embodiments, the battery pack may comprise a plurality of cells, where at least a portion of the plurality of cells are not electrically connected to one another. The battery pack may further comprise a casing defining an interior cavity having at least one interior cavity wall. The at least one interior cavity wall may comprise a first electrode electrically connected to an anode of the battery pack and a second electrode electrically connected to a cathode of the battery pack. The battery pack may further comprise a battery drain positioned within the interior cavity. The battery drain may comprises first and second contacts electrically connected to one another and in contact with the at least one interior cavity wall. The battery drain may be positionable at a first position within the interior cavity where the first and second contacts are not in electrical contact with the first and second electrodes and at a second position where the first contact is in electrical contact with the first electrode and the second contact is in electrical contact with the second electrode.

### DESCRIPTION

Various embodiments are directed to battery powered surgical instruments and batteries comprising features for facilitating shipping, storage and disposal. For example, according to one embodiment, a battery pack may comprise a plurality of cells mechanically and electrically coupled together within a casing having an asymmetric cross-sectional shape. The number and type of cells within the casing may be selected to reduce the power of potential accidental discharges below a threshold level. A surgical instrument for use with the battery pack may comprise a handle defining a plurality of cavities. Each cavity may have an asymmetric cross-sectional shape and at least one of the cavities may have an asymmetric cross-section shape sized to receive the battery pack. An additional cavity and/or cavities may house additional battery packs. According to various embodiments, grouping multiple cells within a single casing may reduce inconveniences associated with loading each cell into the handle individually. At the same time, limiting the number of cells grouped together may reduce safety hazards during shipping, storage and disposal.

According to various embodiments, a surgical instrument may utilize one or more battery packs, each comprising a plurality of cells and at least one switch for electrically connecting the plurality of cells. The switch has an open position, where the cells are electrically disconnected from one another, and a closed position where the cells are electrically connected to one another. The switch may transition from the open position to the closed position when the battery pack is installed in a surgical instrument. In this way, surgical instrument may utilize power associated with a multi-cell battery. At the same time, however, the battery pack may be shipped with the switch in the open position to mitigate the available energy for a short and/or arc and, thereby, mitigate safety hazards during shipping, storage and disposal. In certain embodiments, batteries and cells described herein may have discharge switches for connecting a load across the terminals of the battery or cell to discharge the battery. For example, the discharge switch may be closed prior to disposal. In this way, the battery may discharged either prior to disposal or shortly thereafter. Accordingly, battery safety hazards due to disposal may be mitigated.

Prior to describing embodiments of the cells, batteries, battery packs, and associated surgical instruments, a detailed description of an example embodiments of a battery powered surgical instrument is provided. Although the surgical instruments described herein comprise motorized implements for cutting and stapling, it will be appreciated that the battery configurations described herein may be used with any suitable type of electrical surgical instrument including, for example, cutters, claspers, staplers, RF cutter/coagulators, ultrasonic cutter/coagulators, laser cutter/coagulators, *etc.*

One embodiment of a surgical cutting and fastening instrument is described herein. In general, the embodiments of the instrument described herein are endoscopic surgical cutting and fastening instruments. It should be noted, however, that according to other embodiments, the instrument may be a non-endoscopic surgical cutting and fastening instrument, such as a laparoscopic or open surgical instrument.

In one embodiment the surgical instrument comprises a handle, a shaft, and an articulating end effector pivotally connected to the shaft at an articulation pivot. An articulation control may be provided adjacent to the handle to effect rotation of the end effector about the articulation pivot. In this embodiment, the end effector is configured to act as an endocutter for clamping, severing and stapling tissue, although, in other embodiments, different types of end effectors may be used, such as end effectors for other types of surgical devices, such as graspers, cutters, staplers, clip appliers, access devices, drug/gene therapy devices, ultrasound, RF or laser devices, *etc.*

The handle of the instrument may include a closure trigger and a firing trigger for actuating the end effector. It will be appreciated that instruments having end effectors directed to different surgical tasks may have different numbers or types of triggers or other suitable controls for operating the end effector. The end effector is separated from the handle by a preferably elongate shaft. In one embodiment, a clinician or operator of the instrument may articulate the end effector relative to the shaft by utilizing the articulation control, as described in more detail in pending U.S. Patent Publication No. US2007/158385 A1 (Application No. 11/329,020), filed January 10, 2006, entitled "Surgical Instrument Having An Articulating End Effector," by Geoffrey C. Hueil et al.

The end effector includes in this example, among other things, a staple channel and a pivotally translatable clamping member, such as an anvil, which are maintained at a spacing that assures effective stapling and severing of tissue clamped in the end effector. The handle includes a pistol grip towards which a closure trigger is pivotally drawn by the clinician to cause clamping or closing of the anvil toward the staple channel of the end effector to thereby clamp tissue positioned between the anvil and channel. The firing trigger is farther outboard of the closure trigger. Once the closure trigger is locked in the closure position as further described below, the firing trigger may rotate slightly toward the pistol grip so that it can be reached by the operator using one hand. Then the operator may pivotally draw the firing trigger toward the pistol grip to cause the stapling and severing of clamped tissue in the end effector. In other embodiments, different types of clamping members besides the anvil could be used, such as, for example, an opposing jaw, *etc.*

It will be appreciated that the terms "proximal" and "distal" are used herein with reference to a clinician gripping the handle of an instrument. Thus, the end effector is distal with respect to the more proximal handle. It will be further appreciated that, for convenience and clarity, spatial terms such as "vertical" and "horizontal" are used herein with respect to the drawings. However, surgical instruments are used in many orientations and positions, and these terms are not intended to be limiting and absolute.

The closure trigger may be actuated first. Once the clinician is satisfied with the positioning of the end effector, the clinician may draw back the closure trigger to its fully closed, locked position proximate to the pistol grip. The firing trigger may then be actuated. The firing trigger returns to the open position when the clinician removes pressure, as described more fully below. A release button on the handle, and in this example, on the pistol grip of the handle, when depressed may release the locked closure trigger.

One embodiment of the end effector is described herein. The end effector may include, in addition to the previously-mentioned channel and anvil, a cutting instrument, a sled, a staple cartridge that is removably seated in the channel, and a helical screw shaft. The cutting instrument may be, for example, a knife. The anvil may be pivotably opened and closed at a pivot point connected to the proximate end of the channel. The anvil may also include a tab at its proximate end that is inserted into a component of the mechanical closure system (described further below) to open and close the anvil. When the closure trigger is actuated, that is, drawn in by a user of the instrument, the anvil may pivot about the pivot point into the clamped or closed position. If clamping of the end effector is satisfactory, the operator may actuate the firing trigger, which, as explained in more detail below, causes the knife and sled to travel longitudinally along the channel, thereby cutting tissue clamped within the end effector. The movement of the sled along the channel causes the staples of the staple cartridge to be driven through the severed tissue and against the closed anvil, which turns the staples to fasten the severed tissue. U.S. Patent No. 6,978,921, entitled "Surgical Stapling Instrument Incorporating An E-Beam Firing Mechanism,", provides more details about such two-stroke cutting and fastening instruments. According to various embodiments, the sled may be an integral part of the cartridge, such that when the knife retracts following the cutting operation, the sled does not retract.

It should be noted that although the embodiments of the instrument described herein employ an end effector that staples the severed tissue, in other embodiments different techniques for fastening or sealing the severed tissue may be used. For example, end effectors that use RF energy or adhesives to fasten the severed tissue may also be used. U.S. Patent No. 5,810,811, entitled "Electrosurgical Hemostatic Device,", discloses a cutting instrument that uses RF energy to fasten the severed tissue. U.S. Patent Publication No. US2007/102453 A1 (Application No. 11/267,811), entitled "Surgical Stapling Instruments Structured For Delivery Of Medical Agents" and U.S. Patent Publication No. US2007/102452 A1 (Application No. 11/267,383), entitled "Surgical Stapling Instruments Structured For Pump-Assisted Delivery Of Medical Agents,", disclose cutting instruments that use adhesives to fasten the severed tissue. Accordingly, although the description herein refers to cutting/stapling operations and the like below, it should be recognized that this is an example embodiment and is not meant to be limiting. Other tissue-fastening techniques may also be used.

In one embodiment, the shaft may include a proximate closure tube and a distal closure tube pivotably linked by a pivot links. The distal closure tube includes an opening into which the tab on the anvil is inserted in order to open and close the anvil, as further described below. Disposed inside the closure tubes, may be a proximate spine tube. Disposed inside the proximate spine tube may be a main rotational (or proximate) drive shaft that communicates with a secondary (or distal) drive shaft via a bevel gear assembly. The secondary drive shaft is connected to a drive gear that engages a proximate drive gear of the helical screw shaft. When the main drive shaft is caused to rotate by actuation of the firing trigger (as explained in more detail below), the bevel gear assembly causes the secondary drive shaft to rotate, which in turn, because of the engagement of the drive gears causes the helical screw shaft to rotate, which causes the knife/sled driving member to travel longitudinally along the channel to cut any tissue clamped within the end effector. The vertical bevel gear may sit and pivot in an opening in the distal end of the proximate spine tube. A distal spine tube may be used to enclose the secondary drive shaft and the drive gears. Collectively, the main drive shaft, the secondary drive shaft, and the articulation assembly (e.g., the bevel gear assembly) are sometimes referred to herein as the "main drive shaft assembly."

A bearing is threaded on the helical drive screw. The bearing is also connected to the knife. When the helical drive screw forward rotates, the bearing traverses the helical drive screw distally, driving the cutting instrument and, in the process, the sled to perform the cutting/stapling operation. The sled may be made of, for example, plastic, and may have a sloped distal surface. As the sled traverses the channel, the sloped forward surface may push up or drive the staples in the staple cartridge through the clamped tissue and against the anvil. The anvil turns the staples, thereby stapling the severed tissue. When the knife is retracted, the knife and sled may become disengaged, thereby leaving the sled at the distal end of the channel.

Because of the lack of user feedback for the cutting/stapling operation, there is a general lack of acceptance among physicians of motor-driven surgical instruments where the cutting/stapling operation is actuated by merely pressing a button. In contrast, various embodiments may provide a motor-driven endocutter with user-feedback of the deployment, force, and/or position of the cutting instrument in the end effector.

In one embodiment there is a motor-driven endocutter, and in particular the handle thereof, that provides user-feedback regarding the deployment and loading force of the cutting instrument in the end effector. In addition, the embodiment may use power provided by the user in retracting the firing trigger to power the device (a so-called "power assist" mode). The handle includes exterior lower side pieces and exterior upper side pieces that fit together to form, in general, the exterior of the handle. A battery, such as a Li ion battery, may be provided in the pistol grip portion of the handle. Although the battery is described as containing a single cell, it will be appreciated that the battery, in some embodiments, may include multiple cells connected together. The battery may power a motor disposed in an upper portion of the pistol grip portion of the handle. According to various embodiments, the motor may be a DC brushed driving motor having a maximum rotation of, approximately, 5000 RPM. The motor may drive a 90° bevel gear assembly comprising a first bevel gear and a second bevel gear. The bevel gear assembly may drive a planetary gear assembly. The planetary gear assembly may include a pinion gear connected to a drive shaft. The pinion gear may drive a mating ring gear that drives a helical gear drum via a drive shaft. A ring may be threaded on the helical gear drum. Thus, when the motor rotates, the ring is caused to travel along the helical gear drum by means of the interposed bevel gear assembly, planetary gear assembly and ring gear.

The handle may also include a run motor sensor in communication with the firing trigger to detect when the firing trigger has been drawn in (or "closed") toward the pistol grip portion of the handle by the operator to thereby actuate the cutting/stapling operation by the end effector. The sensor may be a proportional sensor such as, for example, a rheostat or variable resistor. When the firing trigger is drawn in, the sensor detects the movement, and sends an electrical signal indicative of the voltage (or power) to be supplied to the motor. When the sensor is a variable resistor or the like, the rotation of the motor may be generally proportional to the amount of movement of the firing trigger. That is, if the operator only draws or closes the firing trigger in a little bit, the rotation of the motor is relatively low. When the firing trigger is fully drawn in (or in the fully closed position), the rotation of the motor is at its maximum. In other words, the harder the user pulls on the firing trigger, the more voltage is applied to the motor, causing greater rates of rotation.

The handle may include a middle handle piece adjacent to the upper portion of the firing trigger. The handle also may comprise a bias spring connected between posts on the middle handle piece and the firing trigger. The bias spring may bias the firing trigger to its fully open position. In that way, when the operator releases the firing trigger, the bias spring will pull the firing trigger to its open position, thereby removing actuation of the sensor, thereby stopping rotation of the motor. Moreover, by virtue of the bias spring, any time a user closes the firing trigger, the user will experience resistance to the closing operation, thereby providing the user with feedback as to the amount of rotation exerted by the motor. Further, the operator could stop retracting the firing trigger to thereby remove force from the sensor, to thereby stop the motor. As such, the user may stop the deployment of the end effector, thereby providing a measure of control of the cutting/fastening operation to the operator.

The distal end of the helical gear drum includes a distal drive shaft that drives a ring gear, which mates with a pinion gear. The pinion gear is connected to the main drive shaft of the main drive shaft assembly. In that way, rotation of the motor causes the main drive shaft assembly to rotate, which causes actuation of the end effector, as described above.

The ring threaded on the helical gear drum may include a post that is disposed within a slot of a slotted arm. The slotted arm has an opening its opposite end that receives a pivot pin that is connected between the handle exterior side pieces. The pivot pin is also disposed through an opening in the firing trigger and an opening in the middle handle piece.

In addition, the handle may include a reverse motor (or end-of-stroke sensor) and a stop motor (or beginning-of-stroke) sensor. In various embodiments, the reverse motor sensor may be a limit switch located at the distal end of the helical gear drum such that the ring threaded on the helical gear drum contacts and trips the reverse motor sensor when the ring reaches the distal end of the helical gear drum. The reverse motor sensor, when activated, sends a signal to the motor to reverse its rotation direction, thereby withdrawing the knife of the end effector following the cutting operation.

The stop motor sensor may be, for example, a normally-closed limit switch. In various embodiments, it may be located at the proximate end of the helical gear drum so that the ring trips the switch when the ring reaches the proximate end of the helical gear drum.

In operation, when an operator of the instrument pulls back the firing trigger, the sensor detects the deployment of the firing trigger and sends a signal to the motor to cause forward rotation of the motor at, for example, a rate proportional to how hard the operator pulls back the firing trigger. The forward rotation of the motor in turn causes the ring gear at the distal end of the planetary gear assembly to rotate, thereby causing the helical gear drum to rotate, causing the ring threaded on the helical gear drum to travel distally along the helical gear drum. The rotation of the helical gear drum also drives the main drive shaft assembly as described above, which in turn causes deployment of the knife in the end effector. That is, the knife and sled are caused to traverse the channel longitudinally, thereby cutting tissue clamped in the end effector. Also, the stapling operation of the end effector is caused to happen in embodiments where a stapling-type end effector is used.

By the time the cutting/stapling operation of the end effector is complete, the ring on the helical gear drum will have reached the distal end of the helical gear drum, thereby causing the reverse motor sensor to be tripped, which sends a signal to the motor to cause the motor to reverse its rotation. This in turn causes the knife to retract, and also causes the ring on the helical gear drum to move back to the proximate end of the helical gear drum.

The middle handle piece includes a backside shoulder that engages the slotted arm. The middle handle piece also has a forward motion stop that engages the firing trigger. The movement of the slotted arm is controlled, as explained above, by rotation of the motor. When the slotted arm rotates CCW as the ring travels from the proximate end of the helical gear drum to the distal end, the middle handle piece will be free to rotate CCW. Thus, as the user draws in the firing trigger, the firing trigger will engage the forward motion stop of the middle handle piece, causing the middle handle piece to rotate CCW. Due to the backside shoulder engaging the slotted arm, however, the middle handle piece will only be able to rotate CCW as far as the slotted arm permits. In that way, if the motor should stop rotating for some reason, the slotted arm will stop rotating, and the user will not be able to further draw in the firing trigger because the middle handle piece will not be free to rotate CCW due to the slotted arm.

Two states of one embodiment of a variable sensor that may be used as the run motor sensor are described herein. The sensor may include a face portion, a first electrode, a second electrode, and a compressible dielectric material (*e.g.,* EAP) between the electrodes. The sensor may be positioned such that the face portion contacts the firing trigger when retracted. Accordingly, when the firing trigger is retracted, the dielectric material is compressed, such that the electrodes are closer together. Since the distance between the electrodes is directly related to the impedance between the electrodes, the greater the distance the more impedance, and the closer the distance the less impedance. In that way, the amount that the dielectric material is compressed due to retraction of the firing trigger is proportional to the impedance between the electrodes, which can be used to proportionally control the motor.

Components of an example closure system for closing (or clamping) the anvil of the end effector by retracting the closure trigger are described herein. In this embodiment, the closure system includes a yoke connected to the closure trigger by a pin that is inserted through aligned openings in both the closure trigger and the yoke. A pivot pin, about which the closure trigger pivots, is inserted through another opening in the closure trigger which is offset from where the pin is inserted through the closure trigger. Thus, retraction of the closure trigger causes the upper part of the closure trigger, to which the yoke is attached via the pin, to rotate CCW. The distal end of the yoke is connected, via a pin, to a first closure bracket. The first closure bracket connects to a second closure bracket. Collectively, the closure brackets define an opening in which the proximate end of the proximate closure tube is seated and held such that longitudinal movement of the closure brackets causes longitudinal motion by the proximate closure tube. The instrument also includes a closure rod disposed inside the proximate closure tube. The closure rod may include a window into which a post on one of the handle exterior pieces, such as exterior lower side piece in the described embodiment, is disposed to fixedly connect the closure rod to the handle. In that way, the proximate closure tube is capable of moving longitudinally relative to the closure rod. The closure rod may also include a distal collar that fits into a cavity in proximate spine tube and is retained therein by a cap.

In operation, when the yoke rotates due to retraction of the closure trigger, the closure brackets cause the proximate closure tube to move distally (*e.g.,* away from the handle end of the instrument), which causes the distal closure tube to move distally, which causes the anvil to rotate about the pivot point into the clamped or closed position. When the closure trigger is unlocked from the locked position, the proximate closure tube is caused to slide proximally, which causes the distal closure tube to slide proximally, which by virtue of the tab being inserted in the window of the distal closure tube, causes the anvil to pivot about the pivot point into the open or unclamped position. In that way, by retracting and locking the closure trigger, an operator may clamp tissue between the anvil and channel, and may unclamp the tissue following the cutting/stapling operation by unlocking the closure trigger from the locked position.

One embodiment of an electrical circuit of the instrument is described herein. When an operator initially pulls in the firing trigger after locking the closure trigger, the sensor is activated, allowing current to flow therethrough. If the normally-open reverse motor sensor switch is open (meaning the end of the end effector stroke has not been reached), current will flow to a single pole, double throw relay. Since the reverse motor sensor switch is not closed, the coil of the relay will not be energized, so the relay will be in its non-energized state. The circuit also includes a cartridge lockout sensor switch. If the end effector includes a staple cartridge, the sensor switch will be in the closed state, allowing current to flow. Otherwise, if the end effector does not include a staple cartridge, the sensor switch will be open, thereby preventing the battery from powering the motor.

When the staple cartridge is present, the sensor switch is closed, which energizes a single pole, single throw relay. When the relay is energized, current flows through the relay, through the variable resistor sensor, and to the motor via a double pole, double throw relay, thereby powering the motor and allowing it to rotate in the forward direction.

When the end effector reaches the end of its stroke, the reverse motor sensor will be activated, thereby closing the switch and energizing the relay. This causes the relay to assume its energized state, which causes current to bypass the cartridge lockout sensor switch and variable resistor, and instead causes current to flow to both the normally-closed double pole, double throw relay and back to the motor, but in a manner, via the relay, that causes the motor to reverse its rotational direction.

Because the stop motor sensor switch is normally-closed, current will flow back to the relay to keep it energized until the switch opens. When the knife is fully retracted, the stop motor sensor switch is activated, causing the switch to open, thereby removing power from the motor.

In other embodiments, rather than a proportional-type sensor, an on-off type sensor could be used. In such embodiments, the rate of rotation of the motor would not be proportional to the force applied by the operator. Rather, the motor would generally rotate at a constant rate. But the operator would still experience force feedback because the firing trigger is geared into the gear drive train.

A handle of a power-assist motorized endocutter according to another embodiment is described herein. This embodiment is similar to that of previous embodiments except that in this embodiment, there is no slotted arm connected to the ring threaded on the helical gear drum. Instead, the ring includes a sensor portion that moves with the ring as the ring advances down (and back) on the helical gear drum. The sensor portion includes a notch. The reverse motor sensor may be located at the distal end of the notch and the stop motor sensor may be located at the proximate end of the notch. As the ring moves down the helical gear drum (and back), the sensor portion moves with it. Further, the middle piece may have an arm that extends into the notch.

In operation, as an operator of the instrument retracts in the firing trigger toward the pistol grip, the run motor sensor detects the motion and sends a signal to power the motor, which causes, among other things, the helical gear drum to rotate. As the helical gear drum rotates, the ring threaded on the helical gear drum advances (or retracts, depending on the rotation). Also, due to the pulling in of the firing trigger, the middle piece is caused to rotate CCW with the firing trigger due to the forward motion stop that engages the firing trigger. The CCW rotation of the middle piece cause the arm to rotate CCW with the sensor portion of the ring such that the arm stays disposed in the notch. When the ring reaches the distal end of the helical gear drum, the arm will contact and thereby trip the reverse motor sensor. Similarly, when the ring reaches the proximate end of the helical gear drum, the arm will contact and thereby trip the stop motor sensor. Such actions may reverse and stop the motor, respectively, as described above.

The handle of a power-assist motorized endocutter according to another embodiment is described herein. This embodiment is similar to that of previous embodiments except that in this embodiment there is no slot in the arm. Instead, the ring threaded on the helical gear drum includes a vertical channel. Instead of a slot, the arm includes a post that is disposed in the channel. As the helical gear drum rotates, the ring threaded on the helical gear drum advances (or retracts, depending on the rotation). The arm rotates CCW as the ring advances due to the post being disposed in the channel.

As mentioned above, in using a two-stroke motorized instrument, the operator first pulls back and locks the closure trigger. One embodiment of a closure trigger locking mechanism for locking the closure trigger to the pistol grip portion of the handle is described herein. In the described embodiment, the pistol grip portion includes a hook that is biased to rotate CCW about a pivot point by a torsion spring. Also, the closure trigger includes a closure bar. As the operator draws in the closure trigger, the closure bar engages a sloped portion of the hook, thereby rotating the hook upward (or CW) until the closure bar completely passes the sloped portion into a recessed notch of the hook, which locks the closure trigger in place. The operator may release the closure trigger by pushing down on a slide button release on the back or opposite side of the pistol grip portion. Pushing down the slide button release rotates the hook CW such that the closure bar is released from the recessed notch.

Another closure trigger locking mechanism according to various embodiments is described herein. In this embodiment, the closure trigger includes a wedge having an arrow-head portion. The arrow-head portion is biased downward (or CW) by a leaf spring. The wedge and leaf spring may be made from, for example, molded plastic. When the closure trigger is retracted, the arrow-head portion is inserted through an opening in the pistol grip portion of the handle. A lower chamfered surface of the arrow-head portion engages a lower sidewall of the opening, forcing the arrow-head portion to rotate CCW. Eventually the lower chamfered surface fully passes the lower sidewall, removing the CCW force on the arrow-head portion, causing the lower sidewall to slip into a locked position in a notch behind the arrow-head portion.

To unlock the closure trigger, a user presses down on a button on the opposite side of the closure trigger, causing the arrow-head portion to rotate CCW and allowing the arrow-head portion to slide out of the opening.

Another embodiment of a closure trigger locking mechanism is described herein. In this embodiment, the closure trigger includes a flexible longitudinal arm that includes a lateral pin extending therefrom. The arm and pin may be made from molded plastic, for example. The pistol grip portion of the handle includes an opening with a laterally extending wedge disposed therein. When the closure trigger is retracted, the pin engages the wedge, and the pin is forced downward (*e.g.,* the arm is rotated CW) by the lower surface of the wedge When the pin fully passes the lower surface, the CW force on the arm is removed, and the pin is rotated CCW such that the pin comes to rest in a notch behind the wedge, thereby locking the closure trigger. The pin is further held in place in the locked position by a flexible stop extending from the wedge.

To unlock the closure trigger, the operator may further squeeze the closure trigger, causing the pin to engage a sloped backwall of the opening, forcing the pin upward past the flexible stop. The pin is then free to travel out an upper channel in the opening such that the closure trigger is no longer locked to the pistol grip portion.

A universal joint ("u-joint") that may be employed at the articulation point of a surgical instrument, such as the instrument, is described herein. The second piece of the u-joint rotates in a horizontal plane in which the first piece lies. The u-joint may be in a linear (180°) orientation and the u-joint may be at approximately a 150° orientation. The u-joint may be used instead of the bevel gears at the articulation point of the main drive shaft assembly to articulate the end effector. A torsion cable may be used in lieu of both the bevel gears and the u-joint to realize articulation of the end effector.

Another embodiment of a motorized, two-stroke surgical cutting and fastening instrument with power assist is described herein. This embodiment is similar to previous embodiments except that instead of the helical gear drum, this embodiment includes an alternative gear drive assembly. This embodiment includes a gear box assembly including a number of gears disposed in a frame, wherein the gears are connected between the planetary gear and the pinion gear at the proximate end of the drive shaft. As explained further below, the gear box assembly provides feedback to the user via the firing trigger regarding the deployment and loading force of the end effector. Also, the user may provide power to the system via the gear box assembly to assist the deployment of the end effector. In that sense, like the embodiments described above, this embodiment is another power assist, motorized instrument that provides feedback to the user regarding the loading force experienced by the cutting instrument.

In this embodiment, the firing trigger includes two pieces: a main body portion and a stiffening portion. The main body portion may be made of plastic, for example, and the stiffening portion may be made out of a more rigid material, such as metal. In this embodiment, the stiffening portion is adjacent to the main body portion, but according to other embodiments, the stiffening portion could be disposed inside the main body portion. A pivot pin may be inserted through openings in the firing trigger pieces and may be the point about which the firing trigger rotates. In addition, a spring may bias the firing trigger to rotate in a CCW direction. The spring may have a distal end connected to a pin that is connected to the pieces of the firing trigger. The proximate end of the spring may be connected to one of the handle exterior lower side pieces.

In this embodiment, both the main body portion and the stiffening portion include gear portions at their upper end portions. The gear portions engage a gear in the gear box assembly, as explained below, to drive the main drive shaft assembly and to provide feedback to the user regarding the deployment of the end effector.

The gear box assembly may include six (6) gears. A first gear of the gear box assembly engages the gear portions of the firing trigger. In addition, the first gear engages a smaller second gear, the smaller second gear being coaxial with a large third gear. The third gear engages a smaller fourth gear, the smaller fourth gear being coaxial with a fifth gear. The fifth gear is a 90° bevel gear that engages a mating 90° bevel gear that is connected to the pinion gear that drives the main drive shaft.

In operation, when the user retracts the firing trigger, a run motor sensor is activated, which may provide a signal to the motor to rotate at a rate proportional to the extent or force with which the operator is retracting the firing trigger. This causes the motor to rotate at a speed proportional to the signal from the sensor. The sensor could be similar to the run motor sensor described above. The sensor could be located in the handle such that it is depressed when the firing trigger is retracted. Also, instead of a proportional-type sensor, an on/off type sensor may be used.

Rotation of the motor causes the bevel gears to rotate, which causes the planetary gear to rotate, which causes, via the drive shaft, the ring gear to rotate. The ring gear meshes with the pinion gear, which is connected to the main drive shaft. Thus, rotation of the pinion gear drives the main drive shaft, which causes actuation of the cutting/stapling operation of the end effector.

Forward rotation of the pinion gear in turn causes the bevel gear to rotate, which causes, by way of the rest of the gears of the gear box assembly, the first gear to rotate. The first gear engages the gear portions of the firing trigger, thereby causing the firing trigger to rotate CCW when the motor provides forward drive for the end effector (and to rotate CCW when the motor rotates in reverse to retract the end effector). In that way, the user experiences feedback regarding loading force and deployment of the end effector by way of the user's grip on the firing trigger. Thus, when the user retracts the firing trigger, the operator will experience a resistance related to the load force experienced by the end effector. Similarly, when the operator releases the firing trigger after the cutting/stapling operation so that it can return to its original position, the user will experience a CW rotation force from the firing trigger that is generally proportional to the reverse speed of the motor.

It should also be noted that in this embodiment the user can apply force (either in lieu of or in addition to the force from the motor) to actuate the main drive shaft assembly (and hence the cutting/stapling operation of the end effector) through retracting the firing trigger. That is, retracting the firing trigger causes the gear portions to rotate CCW, which causes the gears of the gear box assembly to rotate, thereby causing the pinion gear to rotate, which causes the main drive shaft to rotate.

The instrument may further include reverse motor and stop motor sensors. As described above, the reverse motor and stop motor sensors may detect, respectively, the end of the cutting stroke (full deployment of the knife and sled) and the end of retraction operation (full retraction of the knife). A circuit similar to that described above may be used to appropriately power the motor.

Another embodiment of a two-stroke, motorized surgical cutting and fastening instrument with power assist is described herein. This embodiment is similar to previous embodiments except that in this embodiment, the firing trigger includes a lower portion and an upper portion. Both portions are connected to and pivot about a pivot pin that is disposed through each portion. The upper portion includes a gear portion that engages the first gear of the gear box assembly. The spring is connected to the upper portion such that the upper portion is biased to rotate in the CW direction. The upper portion may also include a lower arm that contacts an upper surface of the lower portion of the firing trigger such that when the upper portion is caused to rotate CW the lower portion also rotates CW, and when the lower portion rotates CCW the upper portion also rotates CCW. Similarly, the lower portion includes a rotational stop that engages a lower shoulder of the upper portion. In that way, when the upper portion is caused to rotate CCW the lower portion also rotates CCW, and when the lower portion rotates CW the upper portion also rotates CW.

This embodiment also includes the run motor sensor that communicates a signal to the motor that, in various embodiments, may cause the motor to rotate at a speed proportional to the force applied by the operator when retracting the firing trigger. The sensor may be, for example, a rheostat or some other variable resistance sensor, as explained herein. In addition, the instrument may include a reverse motor sensor that is tripped or switched when contacted by a front face of the upper portion of the firing trigger. When activated, the reverse motor sensor sends a signal to the motor to reverse direction. Also, the instrument may include a stop motor sensor that is tripped or actuated when contacted by the lower portion of the firing trigger. When activated, the stop motor sensor sends a signal to stop the reverse rotation of the motor.

In operation, when an operator retracts the closure trigger into the locked position, the firing trigger is retracted slightly (through mechanisms known in the art, including U.S. Patent No. 6,978,921, entitled "Surgical Stapling Instrument Incorporating An E-Beam Firing Mechanism" and U.S. Patent No. 6,905,057, entitled "Surgical Stapling Instrument Incorporating A Firing Mechanism Having A Linked Rack Transmission,") so that the user can grasp the firing trigger to initiate the cutting/stapling operation. At that point the gear portion of the upper portion of the firing trigger moves into engagement with the first gear of the gear box assembly. When the operator retracts the firing trigger, according to various embodiments, the firing trigger may rotate a small amount, such as five degrees, before tripping the run motor sensor. Activation of the sensor causes the motor to forward rotate at a rate proportional to the retraction force applied by the operator. The forward rotation of the motor causes, as described above, the main drive shaft to rotate, which causes the knife in the end effector to be deployed (*e.g.,* begin traversing the channel). Rotation of the pinion gear, which is connected to the main drive shaft, causes the gears in the gear box assembly to rotate. Since the first gear is in engagement with the gear portion of the upper portion of the firing trigger, the upper portion is caused to rotate CCW, which causes the lower portion to also rotate CCW.

When the knife is fully deployed (*e.g.,* at the end of the cutting stroke), the front face of the upper portion trips the reverse motor sensor, which sends a signal to the motor to reverse rotational direction. This causes the main drive shaft assembly to reverse rotational direction to retract the knife. Reverse rotation of the main drive shaft assembly causes the gears in the gear box assembly to reverse direction, which causes the upper portion of the firing trigger to rotate CW, which causes the lower portion of the firing trigger to rotate CW until the front face of the upper portion trips or actuates the stop motor sensor when the knife is fully retracted, which causes the motor to stop. In that way, the user experiences feedback regarding deployment of the end effector by way of the user's grip on the firing trigger. Thus, when the user retracts the firing trigger, the operator will experience a resistance related to the deployment of the end effector and, in particular, to the loading force experienced by the knife. Similarly, when the operator releases the firing trigger after the cutting/stapling operation so that it can return to its original position, the user will experience a CW rotation force from the firing trigger that is generally proportional to the reverse speed of the motor.

It should also be noted that in this embodiment the user can apply force (either in lieu of or in addition to the force from the motor) to actuate the main drive shaft assembly (and hence the cutting/stapling operation of the end effector) through retracting the firing trigger. That is, retracting the firing trigger causes the gear portion of the upper portion to rotate CCW, which causes the gears of the gear box assembly to rotate, thereby causing the pinion gear to rotate, which causes the main drive shaft assembly to rotate.

The above-described embodiments employed power-assist user feedback systems, with or without adaptive control (*e.g.,* using a sensor outside of the closed loop system of the motor, gear drive train, and end effector) for a two-stroke, motorized surgical cutting and fastening instrument. That is, force applied by the user in retracting the firing trigger may be added to the force applied by the motor by virtue of the firing trigger being geared into (either directly or indirectly) the gear drive train between the motor and the main drive shaft. In other embodiments, the user may be provided with tactile feedback regarding the position of the knife in the end effector, but without having the firing trigger geared into the gear drive train. One embodiment of a motorized surgical cutting and fastening instrument with such a tactile position feedback system is described herein.

In this embodiment, the firing trigger may have a lower portion and an upper portion, similar to the instrument described above. Unlike the above embodiment, however, the upper portion does not have a gear portion that mates with part of the gear drive train. Instead, the instrument includes a second motor with a threaded rod threaded therein. The threaded rod reciprocates longitudinally in and out of the motor as the motor rotates, depending on the direction of rotation. The instrument also includes an encoder that is responsive to the rotations of the main drive shaft for translating the incremental angular motion of the main drive shaft (or other component of the main drive assembly) into a corresponding series of digital signals, for example. In the described embodiment, the pinion gear includes a proximate drive shaft that connects to the encoder.

The instrument also includes a control circuit, which may be implemented using a microcontroller or some other type of integrated circuit, that receives the digital signals from the encoder. Based on the signals from the encoder, the control circuit may calculate the stage of deployment of the knife in the end effector. That is, the control circuit can calculate if the knife is fully deployed, fully retracted, or at an intermittent stage. Based on the calculation of the stage of deployment of the end effector, the control circuit may send a signal to the second motor to control its rotation to thereby control the reciprocating movement of the threaded rod.

In operation, when the closure trigger is not locked into the clamped position, the firing trigger rotated away from the pistol grip portion of the handle such that the front face of the upper portion of the firing trigger is not in contact with the proximate end of the threaded rod. When the operator retracts the closure trigger and locks it in the clamped position, the firing trigger rotates slightly towards the closure trigger so that the operator can grasp the firing trigger. In this position, the front face of the upper portion contacts the proximate end of the threaded rod.

As the user then retracts the firing trigger, after an initial rotational amount (*e.g.,* 5 degrees of rotation) the run motor sensor may be activated such that, as explained above, the sensor sends a signal to the motor to cause it to rotate at a forward speed proportional to the amount of retraction force applied by the operator to the firing trigger. Forward rotation of the motor causes the main drive shaft to rotate via the gear drive train, which causes the knife and sled to travel down the channel and sever tissue clamped in the end effector. The control circuit receives the output signals from the encoder regarding the incremental rotations of the main drive shaft assembly and sends a signal to the second motor to cause the second motor to rotate, which causes the threaded rod to retract into the motor. This allows the upper portion of the firing trigger to rotate CCW, which allows the lower portion of the firing trigger to also rotate CCW. In that way, because the reciprocating movement of the threaded rod is related to the rotations of the main drive shaft assembly, the operator of the instrument, by way of his/her grip on the firing trigger, experiences tactile feedback as to the position of the end effector. The retraction force applied by the operator, however, does not directly affect the drive of the main drive shaft assembly because the firing trigger is not geared into the gear drive train in this embodiment.

By virtue of tracking the incremental rotations of the main drive shaft assembly via the output signals from the encoder, the control circuit can calculate when the knife is fully deployed (*e.g.,* fully extended). At this point, the control circuit may send a signal to the motor to reverse direction to cause retraction of the knife. The reverse direction of the motor causes the rotation of the main drive shaft assembly to reverse direction, which is also detected by the encoder. Based on the reverse rotation detected by the encoder, the control circuit sends a signal to the second motor to cause it to reverse rotational direction such that the threaded rod starts to extend longitudinally from the motor. This motion forces the upper portion of the firing trigger to rotate CW, which causes the lower portion to rotate CW. In that way, the operator may experience a CW force from the firing trigger, which provides feedback to the operator as to the retraction position of the knife in the end effector. The control circuit can determine when the knife is fully retracted. At this point, the control circuit may send a signal to the motor to stop rotation.

According to other embodiments, rather than having the control circuit determine the position of the knife, reverse motor and stop motor sensors may be used, as described above. In addition, rather than using a proportional sensor to control the rotation of the motor, an on/off switch or sensor can be used. In such an embodiment, the operator would not be able to control the rate of rotation of the motor. Rather, it would rotate at a preprogrammed rate.

Embodiments of batteries and battery configurations for use with powered surgical devices are described herein. The batteries and battery configurations described below may be utilized with any suitable powered surgical instrument including for example, the instrument embodiments described above. In addition to or instead of the functionality of the embodiments described herein above surgical instruments utilizing the batteries and battery configurations described herein may comprise end effectors for cutting, clasping, laser cutting and/or coagulation, RF cutting and/or coagulation, ultrasonic cutting and/or coagulation, *etc.*

One embodiment of a surgical instrument comprising a pair of asymmetrically-shaped battery packs is described herein. The instrument may comprise a handle, a trigger and an end effector. According to various embodiments, the handle, trigger and end effector may operate in a manner similar to that of the various handles, triggers, and end effectors described herein. In addition to or instead of the functionality described herein above, the end effector may comprise surgical implements for cutting, clasping, laser cutting and/or coagulation, RF cutting and/or coagulation, ultrasonic cutting and/or coagulation, *etc.*

The handle of the instrument may house battery packs. The battery packs may be electrically connected to and may provide power to a circuit of the instrument. The circuit may be located in the handle, in the end effector, or in any combination of locations within the instrument. In use, the circuit may power the operation of at least one surgical implement at the end effector. For example, the circuit may comprise an electric motor for operating an electrically powered cutter, clasper, or other mechanical device. In addition to, or instead of a motor, the circuit may comprise suitable circuit components for implementing an RF, ultrasonic, or other type of non-motor-powered surgical implement.

One embodiment of a battery pack outside of the handle is described herein. The battery pack may have an asymmetric cross-sectional shape. In one example the battery pack has a half-ovaloid shape. It will be appreciated that other asymmetric cross-sectional shapes could be used. The battery pack comprises three cells. The cells may be any suitable type of cell including, for example, lithium-ion cells such as the CR123-type cell and/or the CR2-type cell. The cells may be electrically connected to one another in series or parallel. The number of cells may be chosen to the power of any accidental discharge from the battery pack. For example, the number of connected cells may be selected such that the cumulative energy available to an arc or short is less than the energy required to ignite common shipping and/or packing materials. According to various embodiments, this value may be defined by appropriate government regulations.

One embodiment of the handle including cavities for receiving the battery packs is described herein. The cavities may have an asymmetric cross-sectional shape corresponding to the cross-sectional shape of the battery packs. This may allow the battery packs to be received within the cavities. An interior portion of the cavity is also disclosed. A wall may comprise contacts. The contacts may be connected to the circuit and may be configured to connect the battery pack to the circuit when the battery pack is installed in the cavity. It will be appreciated that cavity may comprise a similar interior portion and similar contacts.

One embodiment of the battery pack having a positive electrode contact and a negative electrode contact is described herein. Upon insertion of the battery pack within the cavity, the electrode contacts may connect to the contacts to establish a connection between the battery pack and the circuit. The electrode contacts may be disposed on a first end of the battery pack. It will be appreciated, however, that the electrode contacts may be positioned on any other surface of the battery pack including, for example, the end, flat face and/or curved face. Accordingly, the contacts may be positioned on a corresponding surface of the interior portion of the cavity.

The asymmetric cross sectional shape of the battery packs and the cavities may ensure that the battery packs are inserted into the instrument with the correct polarity. For example, due to its asymmetric cross-sectional shape, the end of the battery pack may fit into the cavity of the handle in only one orientation, ensuring that the correct electrodes are in contact with one another. Similarly, the end of the battery pack may fit into the cavity in only one orientation. Because the cross-sectional shape of the cavity is reciprocal to that of the cavity, the orientation of the electrode contacts may be reversed in the cavity relative to the cavity. Accordingly, when the cavities have reciprocal cross-sectional shapes the position of the contacts within the cavity may also be reversed to ensure correct polarity.

The clinician may be relied upon to recognize that the end of the battery pack with the electrodes is properly inserted into the cavities. According to various embodiments, however, the form of the battery pack may be manipulated to make it difficult or impossible for the end of the battery pack to be inserted into the cavities. For example, the battery pack may include an optional flange at the end. The flange may extend beyond the battery pack to ensure that the end cannot be inserted into one of the cavities. Although the instrument described utilizes two battery packs and defines two cavities, it will be appreciated that more or additional battery packs and corresponding cavities may be used.

One embodiment of the battery pack in conjunction with a discharge plug is described herein. The discharge plug may be attached to the end of the battery pack, for example, after use of the battery pack is complete. In certain embodiments, the discharge plug may have a cross-sectional area slightly larger than that of the battery pack and may slide over the end. The discharge plug may comprise electrode contacts electrically connected to one another via a resistive element. The resistive element may be any suitable resistive element having any suitable electrical resistance and/or impedance. With the discharge in place, the electrode contacts may contact positive and negative electrode contacts. This may place the resistive element in series with the battery pack, causing the battery to drain. In this way, the battery pack may be drained either prior to or during disposal, reducing hazard disposal.

A schematic diagram of one embodiment of a surgical instrument and a battery pack is described herein. The surgical instrument may operate in a manner similar to that of the surgical instruments described herein above. For example, the instrument may be any suitable type of surgical instrument utilizing battery power including, for example, instruments having motorized implements for cutting, motorized implements for stapling, RF implements for cutting and/or coagulating, ultrasonic implements for cutting and/or coagulating, laser implements for cutting/coagulating, *etc.* The surgical instrument may comprise a pair of electrodes, which, when the battery pack is connected to the surgical instrument, may connect with a pair of electrodes of the battery pack.

The battery pack may comprise a plurality of cells. The cells may be any suitable type of cell. According to various embodiments, the cells may be lithium-ion cells such as the CR123-type cell and/or the CR2-type cell. A switch may have an open position and a closed position. The switch may be any suitable type of mechanical or solid state switch. When the switch is in the open position, the cells may be electrically disconnected from one another. When the switch is in the closed position, the cells may be electrically connected to one another. For example, the cells may be connected in parallel. In various embodiments, however, the cells may be connected in series or in any other desirable configuration. The switch may be engaged to the closed position at the time that the battery pack is connected to the surgical instrument. For example, the switch may be manually engaged by a clinician using the surgical instrument either before or after the battery pack is connected to the instrument. Also, according to various embodiments, the switch may be engaged to the closed position automatically when the battery pack is connected to the instrument (*e.g.,* by placing at least a portion of the battery pack within the surgical instrument).

The battery pack may also comprise a discharge system. The discharge system may comprise a discharge switch and a resistive element. The resistive element may be any suitable resistive element having any suitable electrical resistance and/or impedance. The discharge switch may have an open position and a closed position. When the discharge switch is in the open position, the resistive element may not be electrically connected to the battery pack. When the discharge switch is in the closed position, the resistive element may be electrically connected across the cells of the battery pack. In this way, the cells may drain when the discharge switch is closed. The discharge switch may be any type of mechanical or solid state switch. The discharge switch may be manually or automatically transitioned from the open to the closed position, for example, upon installation of the battery pack to the instrument or upon removal of the surgical instrument from the instrument. In some embodiments, the cells may deliver sufficient power and/or the resistive element may be designed such that discharge switch may be closed while the instrument is in use.

An alternate embodiment of the battery pack and surgical instrument is described herein. The switch may comprise at least one open portion and at least one contactor. The at least one contactor may be a part of the surgical instrument. In this way, the cells may be electrically connected to one another when the battery pack is installed to the surgical instrument, bringing the at least one connector portion in electrical contact with the at least one open portion.

Another embodiment of the battery pack is described herein. The switch is implemented with an open portion, a contactor and a movable tab. The contactor may be mechanically biased against the open portion, for example, by a spring. The movable tab may be positioned between the open portion and the contactor. The movable tab may be made from an insulating material, such as plastic. In this way, the cells may not be electrically connected to one another when the movable tab is in place. When the battery pack is ready for use, the tab may be removed, for example, by the clinician. When the tab is removed, the contactor may be mechanically pushed into electrical contact with the open portion, resulting in the electrical connection of the cells to one another. According to various embodiments, the tab may comprise a portion configured to be received by a corresponding portion of the surgical instrument. When the battery pack is installed to the instrument, the portion of the surgical instrument may contact the tab, tending to remove it from between the open portion and the contactor. The tab may be made from a polymer or any suitable electrically insulating material. Also, according to various embodiments, the tab may have a thickness of about 0.0254 mm (1 mil).

One mechanical embodiment of a battery pack implementing the schematic of the battery pack is described herein. The battery pack comprises a casing having therein a battery comprising a plurality of cells that can be interconnected to one another by connecting contacts. A discharge switch may, when in the closed position, connect a resistive element across the terminals of the cells, causing them to discharge. The battery pack may comprise a pair of contacts positioned on a switch platform. The contactors may have an open position and a closed position. In the closed position, the contactors may be placed in electrical contact with the contacts, causing the cells to be interconnected to one another. Collectively, the switch platform, contacts, and contactors may form a switch. According to various embodiment, when the switch is closed (*e.g.,* the contactors are in contact with the contacts), the cells of the battery may electrically interconnected.

The switch platform may be coupled to a clutch comprising a pair of locking mechanisms. In the position the clutch (including locking mechanisms) is engaged, holding the switch platform in the open position. The battery pack may also comprise a discharge switch. In a closed position, the discharge switch may switch a resistive element across the anode and the cathode of the cells causing the cells to discharge. The discharge switch may be mechanically biased to the closed position by a spring. The bias of the spring, however, may be overcome by a stopper in contact with a movable portion or panel of the casing.

A configuration of the battery pack upon insertion into a surgical instrument is described herein. The battery pack may be inserted into a cavity defined by the instrument. The cavity may be positioned at any portion of the instrument including, in various embodiments, at a handle portion. The cavity may comprise a pair of contacts that may be aligned with contactors. The battery pack may be inserted into the instrument. As the battery pack is inserted, contactors may come into contact with the contacts. This may force the contactors, and the switch platform toward the contacts such that the contactors are in electrical communication with the contacts and the contacts, which may cause the cells of the battery to be interconnected and connected to the instrument.

According to various embodiments, pressure from the contacts may overcome the force of the clutch, disengaging the lock mechanisms, allowing the switch platform to translate towards the contacts. Also, in various embodiments, a portion of an interior of the cavity may comprise one or more keyed portions that are aligned with one or more receptacles associated (*e.g.,* mechanically or electronically) with the lock mechanisms. When the keyed portions come into contact with the receptacles, the clutch lock mechanisms may be enabled to disengage, allowing the switch platform to assume a position. According to various embodiments, after the switch platform assumes the position, the lock mechanisms may reengage, locking the switch platform in place. According to various embodiments, this may make it difficult for the battery pack to lose electrical connectivity with the instrument after insertion.

The interior of the cavity may also comprise a feature (*e.g.,* an extension), for contacting the panel. For example, as the battery pack is inserted into the cavity, the extension may contact the panel, sliding it in the direction of arrow and allowing the stopper to protrude through the casing (*e.g.,* because of the biasing of the spring). According to various embodiments, the stopper may contact the interior wall of the cavity, preventing the discharge switch from being closed. One embodiment of the battery pack after removal from the surgical instrument is described herein. The switch platform may be locked by the lock mechanisms into the same position described above. Also, within the interior wall of the cavity, the stopper may protrude from the casing by an amount suitable to close the discharge switch. This may cause the battery to discharge.

Another mechanical embodiment of a battery pack implementing the schematic of the battery pack is described herein. The battery pack may comprise a casing defining an interior cavity. The casing may be covered by a cap that may be secured to the casing utilizing one or more mechanical latches. One embodiment of the battery pack with the cap removed to show a plurality of cells within is described herein. Any suitable number and/or type of cells may be used. For example, CR123 and/or CR2 cells may be used. One embodiment of the battery pack with a portion of the casing removed to reveal the cells is described herein.

A cross-sectional view of one embodiment of the battery pack including a battery drain is described herein. The battery drain may be positioned within the interior cavity and may be slidable within the interior cavity in the directions of arrow. The drain may comprise at least two contacts. A portion of the contacts may touch wall of the interior cavity. According to various embodiments, the contacts may be biased to exert a force against the walls in order to resist movement of the drain in the direction of the arrows. Also, in some embodiments, the walls may define one or more protrusions or catch members shaped to be received by a portion of one or more of the contacts to hold the drain at a first position. Additionally, the walls may define one or more electrodes. The electrodes may be wired to the cells, such that making an electrical connection across the electrodes may short the positive and negative electrodes of the cells.

The contacts of the drain may be coupled at a base portion of the drain. According to various embodiments, the contacts may be electrically shorted to one another, or may be electrically connected to one another via a resistive element. One embodiment of the battery pack being installed to a surgical instrument is described herein. The surgical instrument may comprise an extending member configured to be received into the interior cavity. The extending member may comprise one or more electrodes positioned to contact electrodes of the battery pack when the member is completely installed. In this way, the cells of the battery pack may provide electrical power to the instrument via the electrodes.

As the member is inserted into the interior cavity, it may contact the battery drain and force it along the interior cavity in the direction of the arrow. For example, the force provided to the battery drain by the member may overcome the drain's resistance to movement provided by the contacts, for example, in conjunction with the catch members. When completely installed the member may push the drain into the cavity until the contacts come into electrical contact with the electrodes. This may either short the cells or electrically connect them across the resistive element. When the battery pack is uninstalled from the instrument, the member may be removed from the cavity. The drain, however, may remain in position. In this way, the cells may drain any remaining charge across the resistive element either before or during disposal. This may, for example, minimize the power of any accidental discharges during disposal.

One embodiment of the battery drain removed from the casing is described herein. The drain may comprise two sets of contacts. The base may define a central portion between the two sets of contacts. According to various embodiments, the central portion may be configured to contact the member. Resistive elements may be mounted to the base. The resistive elements may be elements of any suitable resistance value and any suitable mechanical configuration. For example the resistive elements may comprise one or more surface-mount components.

It is to be understood that at least some of the descriptions herein have been simplified to illustrate elements that are relevant for a clear understanding of the disclosure, while eliminating, for purposes of clarity, other elements. Those of ordinary skill in the art will recognize, however, that these and other elements may be desirable. However, because such elements are well known in the art, and because they do not facilitate a better understanding of the disclosure, a discussion of such elements is not provided herein.

While several embodiments have been described, it should be apparent, however, that various modifications, alterations and adaptations to those embodiments may occur to persons skilled in the art with the attainment of some or all of the advantages of the disclosure. For example, according to various embodiments, a single component may be replaced by multiple components, and multiple components may be replaced by a single component, to perform a given function or functions. This application is therefore intended to cover all such modifications, alterations and adaptations without departing from the scope of the disclosure as defined by the appended claims.

## Claims

1. A surgical system comprising:
a battery pack, wherein the battery pack comprises:
a plurality of cells;
a casing defining an interior cavity having at least one interior cavity wall, wherein the at least one interior cavity wall comprises a first electrode electrically connected to an anode of the battery pack and a second electrode electrically connected to a cathode of the battery pack;
a battery drain positioned within the interior cavity, wherein the battery drain comprises first and second contacts electrically connected to one another, wherein the first and second contacts are in contact with the at least one interior cavity wall, and wherein the battery drain is positionable at a first position within the interior cavity where the first and second contacts are not in electrical contact with the first and second electrodes and a second position where the first contact is in electrical contact with the first electrode and the second contact is in electrical contact with the second electrode; and
a switch having an open position and a closed position, wherein when the switch is in its open position at least a portion of the plurality of cells are not electrically connected to one another, and wherein when the switch is in its closed position the plurality of cells are electrically connected to one another.

2. The surgical system of claim 1, wherein the first and second contacts are electrically connected to one another via a resistive element.

3. The surgical system of claim 1, wherein the battery pack further comprises at least one catch member positioned to engage at least one of the first contact and the second contact to hold the battery drain at the first position.

4. The surgical system of claim 1, further comprising:
an end effector;
a handle operatively coupled to the end effector, wherein the handle comprises a trigger to actuate the end effector, wherein the handle defines a cavity for receiving the battery pack, wherein the handle comprises a connector positioned to connect the plurality of cells to one another when the battery pack is inserted into the cavity, and wherein the handle defines an extension member positioned to contact the battery drain as the battery pack is inserted into the cavity to push the battery drain to the second position.

5. The surgical system of claim 4, wherein the end effector comprises at least one implement selected from the group consisting of: a cutter, a clasper, a stapler, an RF sealing implement, and an ultrasonic implement.

6. The surgical system of claim 1, wherein the plurality of cells comprises at least one cell selected from the group consisting of a CR123 cell and a CR2 cell.

## Patentansprüche

1. Chirurgisches System, umfassend:
ein Batteriepack, wobei das Batteriepack umfasst:
eine Vielzahl von Zellen;
ein Gehäuse, das einen Innenhohlraum mit mindestens einer Innenhohlraumwand definiert, wobei die mindestens eine Innenhohlraumwand eine erste Elektrode umfasst, die elektrisch mit einer Anode des Batteriepacks verbunden ist, und eine zweite Elektrode, die elektrisch mit einer Kathode des Batteriepacks verbunden ist;
eine Leistungsentnahme, die in dem Innenhohlraum positioniert ist, wobei die Leistungsentnahme einen ersten und einen zweiten Kontakt umfasst, die elektrisch miteinander verbunden sind, wobei der erste und der zweite Kontakt in Kontakt mit der mindestens einen Innenhohlraumwand sind, und wobei die Leistungsentnahme in einer ersten Position in dem Innenhohlraum positionierbar ist, in der der erste und der zweite Kontakt nicht in elektrischem Kontakt mit der ersten und der zweiten Elektrode sind, und in einer zweiten Position, in der der erste Kontakt mit der ersten Elektrode in elektrischem Kontakt ist und der zweite Kontakt mit der zweiten Elektrode in elektrischem Kontakt ist; und
einen Schalter mit einer offenen Position und einer geschlossenen Position, wobei, wenn der Schalter in seiner offenen Position ist, mindestens ein Abschnitt der Vielzahl von Zellen nicht elektrisch miteinander verbunden ist, und wobei, wenn der Schalter in seiner geschlossenen Position ist, die Vielzahl von Zellen elektrisch miteinander verbunden sind.

2. Chirurgisches System nach Anspruch 1, wobei der erste und der zweite Kontakt über ein resistives Element elektrisch miteinander verbunden sind.

3. Chirurgisches System nach Anspruch 1, wobei das Batteriepack ferner mindestens ein Fangelement umfasst, das dazu positioniert ist, mindestens einen des ersten Kontakts und des zweiten Kontakts in Eingriff zu bringen, um die Leistungsentnahme in der ersten Position zu halten.

4. Chirurgisches System nach Anspruch 1, ferner umfassend:
einen Endeffektor;
einen Griff, der funktionell mit dem Endeffektor gekoppelt ist, wobei der Griff einen Auslöser umfasst, um den Endeffektor zu betätigen, wobei der Griff einen Hohlraum zum Aufnehmen des Batteriepacks definiert, wobei der Griff einen Verbinder umfasst, der dazu positioniert ist, die Vielzahl von Zellen miteinander zu verbinden, wenn das Batteriepack in den Hohlraum eingesetzt wird, und wobei der Griff ein Verlängerungselement definiert, das dazu positioniert ist, die Leistungsentnahme zu positionieren, wenn das Batteriepack in den Hohlraum eingesetzt wird, um die Leistungsentnahme in die zweite Position zu drücken.

5. Chirurgisches System nach Anspruch 4, wobei der Endeffektor mindestens ein Werkzeug umfasst, ausgewählt aus der Gruppe bestehend aus: einem Schneidwerkzeug, einem Klammerwerkzeug, einem Klammernahtgerät, einem HF-Dichtwerkzeug und einem Ultraschallwerkzeug.

6. Chirurgisches System nach Anspruch 1, wobei die Vielzahl von Zellen mindestens eine Zelle umfasst, die aus der Gruppe bestehend aus einer CR123-Zelle und einer CR2-Zelle besteht.

## Revendications

1. Système chirurgical comprenant :
un bloc-batterie, le bloc-batterie comprenant :
une pluralité de piles ;
un boîtier définissant une cavité intérieure ayant au moins une paroi de cavité intérieure, l'au moins une paroi de cavité intérieure comprenant une première électrode reliée électriquement à une anode du bloc-batterie et une deuxième électrode reliée électriquement à une cathode du bloc-batterie ;
un drain de batterie positionné à l'intérieur de la cavité intérieure, le drain de batterie comprenant des premier et deuxième contacts reliés électriquement l'un à l'autre, les premier et deuxième contacts étant en contact avec l'au moins une paroi de cavité intérieure, et le drain de batterie étant positionnable à une première position à l'intérieur de la cavité intérieure où les premier et deuxième contacts ne sont pas en contact électrique avec les première et deuxième électrodes et une deuxième position où le premier contact est en contact électrique avec la première électrode et le deuxième contact est en contact électrique avec la deuxième électrode ; et
un commutateur ayant une position ouverte et une position fermée, dans lequel, quand le commutateur est dans sa position ouverte, au moins une partie de la pluralité de piles ne sont pas reliées électriquement les unes aux autres, et dans lequel, quand le commutateur est dans sa position fermée, la pluralité de piles sont reliées électriquement les unes aux autres.

2. Système chirurgical de la revendication 1, dans lequel les premier et deuxième contacts sont reliés électriquement l'un à l'autre par le biais d'un élément résistif.

3. Système chirurgical de la revendication 1, dans lequel le bloc-batterie comprend en outre au moins un élément de prise positionné pour s'enclencher avec le premier contact et/ou le deuxième contact pour maintenir le drain de batterie à la première position.

4. Système chirurgical de la revendication 1, comprenant en outre :
un effecteur terminal ;
une poignée fonctionnellement couplée à l'effecteur terminal, la poignée comprenant une gâchette pour actionner l'effecteur terminal, la poignée définissant une cavité destinée à recevoir le bloc-batterie, la poignée comprenant un connecteur positionné pour relier la pluralité de piles les unes aux autres quand le bloc-batterie est inséré dans la cavité, et la poignée définissant un élément de prolongement positionné pour entrer en contact avec le drain de batterie lorsque le bloc-batterie est inséré dans la cavité pour pousser le drain de batterie jusqu'à la deuxième position.

5. Système chirurgical de la revendication 4, dans lequel l'effecteur terminal comprend au moins un instrument choisi dans le groupe constitué par : un instrument tranchant, un instrument de serrage, une agrafeuse, un instrument de fermeture RF, et un instrument ultrasonore.

6. Système chirurgical de la revendication 1, dans lequel la pluralité de piles comprend au moins une pile choisie dans le groupe constitué par une pile CR123 et une pile CR2.
